# EUROPEAN PATENT SPECIFICATION

(11) **EP 3 801 539 B1**
(45) Date of publication and mention of the grant of the patent: **01.04.2026**
(21) Application number: 19831307.4
(22) Date of filing: 30.05.2019
(51) Int. Cl.: A61K 9/20, A61K 31/522, A61P 3/10

(54) **SOLID ORAL PHARMACEUTICAL COMPOSITIONS OF LINAGLIPTIN**
FESTE ORALE PHARMAZEUTISCHE ZUSAMMENSETZUNGEN VON LINAGLIPTIN
COMPOSITOINS PHARMACEUTIQUES SOLIDES D'ADMINISTRATION PAR VOIE ORALE COMPRENANT DE LA LINAGLIPTINE

(30) Priority: 01.06.2018 TR 201807846
(43) Date of publication of application: 14.04.2021
(73) Proprietor: Sanovel Ilac Sanayi ve Ticaret A.S., Sisli - Istanbul (TR)
(72) Inventor: TURKYILMAZ, Ali, 34460 Istanbul (TR); PEHLIVAN AKALIN, Nur, 34460 Sar yer/Istanbul (TR); OZDEN, Aydan, 34460 Sar yer/Istanbul (TR)
(74) Representative: Genç Ilhan, Oznur
(86) International application number: PCT/TR2019/050397
(87) International publication number: WO 2020/009675

(56) References cited:
- EP-A1- 1 852 108
- WO-A1-2015/110962
- WO-A1-2017/060398
- US-A1- 2014 100 236

## Description

### Field of Invention

The present invention relates to solid oral pharmaceutical compositions comprising linagliptin or a pharmaceutically acceptable salt thereof which provide enhanced stability.

### Background of Invention

Linagliptin is used for type 2 or non-insulin dependent diabetes. It is a selective, orally administered, xanthine based dipeptidyl peptidase-4 (DPP-4) inhibitor used as an adjunct to diet and exercise to improve glycemic control. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Linagliptin works by binding to DPP-4 and preventing it from breaking down the GLP-1 and GIP. This increases the levels of these hormones in the body and so increases their effect on controlling blood sugar.

Its chemical name is 8-[(3R)-3-aminopiperidin-1-yl]-7-but-2-yn-1-yl)-3-methyl-1-[(4-methylquinazolin-2-yl)methyl]-3,7-dihydro-1H-purine-2,6-dione and its chemical structure is shown in the Formula I.

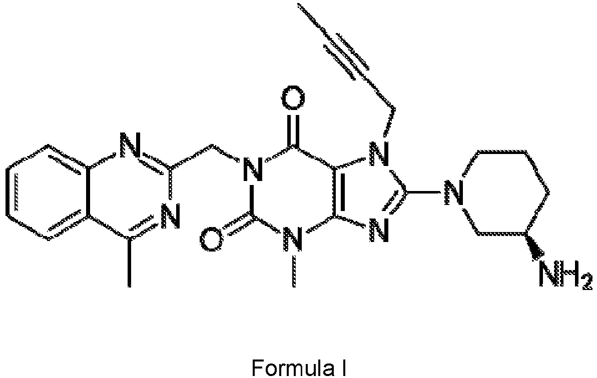

It is used once daily and can be used either alone or in combination with insulin or other glycemic agents. The currently commercially available dose of linagliptin is 5 mg and due to the low active substance content in the final formulation, the active substance is milled in order to ensure an adequate content uniformity.

EP1786401B1, a patent document in the prior art, discloses moisture-sensitive dosage forms comprising a DPP4 inhibitor. In the document, microcrystalline cellulose is suggested as diluent and stearates such as magnesium stearate and calcium stearate are considered as lubricant. It is a fact that diluents are in relatively high amounts in solid dosage forms considering other excipients. For this document, this amount is in between 15-40% by weight of the composition which obviously plays a significant role in the characteristics of the dosage form. However, microcrystalline cellulose is a hygroscopic material (Handbook of Pharmaceutical Excipients edited by Raymond C. Rowe, Paul J. Sheskey and Marlan E. Quinn, sixth edition, page 131) and this range of amount shall not provide the promised moisture resistance for the dosage form. Another issue is that DPP-4 inhibitors with primary amine group show incompatibilities, degradation problems or extraction problems with excipients such as microcrystalline cellulose and stearate lubricants (Journal of Applied Pharmaceutical Science, 2011, Nishath Fathima, Tirunagari Mamatha, Husna Kanwal Qureshi, Nandagopal Anitha and Jangala Venkateswara Rao). Linagliptin has also a primary amine group on its chemical structure; although it is very stable by itself, it may react with these excipients or impurities of these excipients.

Another patent document numbered EP2023902B1 pretends to solve the above-mentioned incompatibility problems and accordingly suggests diluents other than microcrystalline cellulose such as mannitol and pregelatinized starch. Additionally, the suggested formulation comprises corn starch as disintegrant and magnesium stearate as lubricant. The amount of pregelatinized is at least 9% and tends to increase to 20% by weight of the total composition. On the other part, corn starch is present in the amount of 10% by weight in the composition. However, starch and magnesium stearate are also known as excipient which make high impurities and which make incompatibility problems. Moreover, they are also highly hygroscopic. And the mentioned amounts of these excipients are obviously risky to provide stability of a solid dosage form. There is also another issue that the increase of the magnesium stearate amount shall reduce the dissolution rate and the promised enhanced bioavailability accordingly (Handbook of Pharmaceutical Excipients, sixth edition, page 405).

WO 2017/060398 A1 discloses linagliptin formulations which are free of binder. According to WO 2017/060398 A1, linagliptin formulations are prepared by direct compression method. It is mentioned in WO 2017/060398 A1 that, the overall production costs for the pharmaceutical composition are minimized and the omission of the binder combined with the use of direct compression for manufacturing the pharmaceutical composition neither negatively influences the dissolution rate nor the content uniformity and allows high speed industrial manufacturing, thus permitting to produce the pharmaceutical composition at low costs. In another embodiment of WO 2017/060398 A1; the use of disintegrant is also excluded in linagliptin formulations.

It can be seen that there is no teaching, suggestion or motivation in the prior art about how to overcome stability problem and there is still a need for a solid oral pharmaceutical formulation eliminating both incompatibility and hygroscopicity problems at the same time.

### Objects and Brief Description of the Invention

The main object of the present invention is to obtain solid oral pharmaceutical compositions of linagliptin eliminating all aforesaid problems and bringing additional advantages to the relevant prior art.

Another object of the present invention is to develop solid oral pharmaceutical compositions of linagliptin with enhanced stability.

Another object of the present invention is to obtain tablet compositions of linagliptin with increased uniformity.

Another object of the present invention is to obtain tablet compositions of linagliptin with enhanced dissolution profile and bioavailability.

A further object of the present invention is to develop tablet compositions of linagliptin which is free of microcrystalline cellulose, croscarmellose sodium and stearates.

A further object of the present invention is to develop tablet compositions of linagliptin which comprise starch in the amount of less than 10% by weight of the total composition.

Yet another object of the present invention is to improve a process (not claimed) for preparing the said linagliptin tablet compositions, comprising wet granulation method which makes round and compact granules having a high content uniformity and a narrow range of particle sizes.

### Description of the invention

In accordance with the objects outlined above, detailed features of the present invention are given herein.

The present invention relates to a solid oral pharmaceutical formulation comprising linagliptin or a pharmaceutically acceptable salt thereof, wherein the composition is free of microcrystalline cellulose, croscarmellose sodium and stearates; wherein the total amount of starch in the composition is less than 5% by weight of the total composition and wherein the solid oral pharmaceutical formulation further comprises polyvinyl alcohol in an amount of between 0.1-10% by weight of the total formulation.

They are all hygroscopic excipients with impurities such as antioxidants and acids which may interact with primary amine groups, cause incompatibility problems and reduce stability accordingly.

According to the preferred embodiment, the total amount of starch in the composition is less than 10%, preferably less than 5% by weight of the total composition, since they also tend to increase hygroscopic behavior of the composition in high amounts. According to the preferred embodiment, the composition comprises linagliptin free base. The amount of linagliptin in an amount of 0.1-15%, preferably 0.5-10%, more preferably 1-5% by weight of the composition.

The composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet or orally administrable film.

According to the preferred embodiment, the form of the composition is film-coated tablet.

According to one embodiment, the composition further comprises at least one pharmaceutically acceptable excipient selected from diluents, disintegrants, binders, lubricants or mixtures thereof.

According to one embodiment, the solid oral pharmaceutical composition comprises at least one diluent which is selected from the group comprising lactose monohydrate, lactose, dibasic calcium phosphate, mannitol, spray-dried mannitol, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to the preferred embodiment, the solid oral pharmaceutical composition comprises one diluent which is mannitol.

Mannitol is a nonhygroscopic material and is thus suitable for moisture-sensitive compositions. The amount of mannitol is between 20-95%, preferably 40-90% and more preferably 70-85% by weight of the total composition.

According to one embodiment, the solid oral pharmaceutical composition comprises at least one disintegrant which is selected from the group comprising sodium starch glycolate, sodium carbonate, hydroxylpropyl cellulose (HPC), cross-linked polyvinylpyrrolidone (crospovidon), copovidon, polycarbophil, low-substitue poloxamer, starch, pregelatinized starch, alginic acid and alginates, ion-exchange resins, magnesium aluminum silica, sodium dodecyl sulphate, sodium carboxy methyl cellulose, carboxy methyl cellulose calcium, docusate sodium, guar gum, polyacrylin potasium, sodium alginate, sodium glysin carbonate, sodium lauryl sulphate or mixtures thereof.

According to the preferred embodiment, the solid oral pharmaceutical composition comprises one disintegrant which is sodium starch glycolate. The amount of sodium starch glycolate is less than 10%, preferably less than 5% by weight of the total composition.

Starch and its derivatives are known as hygroscopic materials and they have also impurities such as lignin and hemicelluloses which may cause incompatibility problems in the case of overuse regarding the potential interactions with linagliptin. The amount of sodium starch glycolate is thus kept under the risky value for this composition.

According to one embodiment, the solid oral pharmaceutical composition comprises at least one binder which is selected from the group comprising copovidone, copolyvidone, polyvinylpyrrolidone (PVP), povidone, carnauba wax, hydroxypropyl methyl cellulose (HPMC), pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose, polymetacrylates, polyethylene oxide, polyvinyl alcohol (PVA), polycarbophil, polyvinyl acetate and its copolymers, gelatin, pregelatinized starch, xanthan gum, guar gum, alginate, carrageen, collagen, agar, pectin, hyaluronic acid, carbomer, cellulose acetate phthalate, hydroxypropyl starch, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

According to the preferred embodiment, the solid oral pharmaceutical composition comprises one binder which is povidone or polyvinyl alcohol. The claims demand the presence of polyvinyl alcohol. In one embodiment, the amount of povidone is less than 10%, preferably less than 5%, more preferably less than 3% by weight of the total composition. According to the claims, the amount of polyvinyl alcohol is between 0.1-10%, preferably 1-5% by weight of the total composition.

According to one embodiment, the solid oral pharmaceutical composition comprises at least one lubricant which is selected from the group comprising sodium lauryl sulphate, sodium stearyl fumarate, mineral oil, talc, polyethylene glycol, magnesium lauryl sulphate, fumaric acid, stearic acid, hydrogenated natural oils, silica, paraffin or mixtures thereof.

According to the preferred embodiment, the solid oral pharmaceutical composition comprises one lubricant which is sodium stearyl fumarate. The amount of sodium stearyl fumarate is between 0.1-10%, preferably 1-5% by weight of the total composition.

According to the preferred embodiment, the solid oral pharmaceutical composition is free of glidant.

It has been surprisingly found that the use of mannitol as diluent gives softer granules compared to the diluents used in the prior art such as microcrystalline cellulose or dextrose or sucrose. The presence of glidant is not necessary for the suggested composition, since the interparticle friction is significantly reduced. Accordingly, the use of magnesium stearate, which is highly preferable in the prior art based on its combined effect as lubricant and glidant, can also be ruled out and it is thus possible to eliminate its negative effects on the stability of the solid dosage form.

According to one embodiment, the solid oral pharmaceutical composition comprises at least one coating layer to protect the composition against the moisture and maintain the stability. Suitable coating ingredients are selected from the group comprising hydroxypropylmethyl cellulose (hypromellose), hydroxypropyl cellulose, polyvinyl alcohol (PVA), polyethylene glycol (PEG), talc, polyvinyl alcohol-polyethylene glycol copolymers (Kollicoat IR), ethylcellulose dispersions (Surelease), polyvinylprolidone, polyvinylprolidone-vinyl acetate copolymer (PVP-VA) and all kinds of Opadry^{®}, pigments, dyes, titanium dioxide, iron oxide or polymethylmetacrylate copolymers (Eudragit) and mixtures thereof.

According to the preferred embodiment, coating layer is Opadry^{®} which comprises hypromellose, polyethylene glycol, titanium dioxide, glycerin, at least one pigment or dye.

The amount of the coating is between 1-10%, preferably 1-6% by weight of the total composition.

Disclosed, but not claimed, is that the solid oral pharmaceutical composition comprises;
- 0.1-15% by weight of linagliptin,
- 20-95% by weight of mannitol,
- less than 10% by weight of sodium starch glycolate,
- less than 10% by weight of povidone,
- 0.1-10% by weight of sodium stearyl fumarate,
- 1-10% by weight of film coating.

These analytically selected excipients and ratios ensure the required effective doses for the treatment and enhance stability and dissolution profile of the film-coated tablet subjected to the invention.

According to all these embodiments, the below given formulations can be used in the solid oral pharmaceutical composition subjected to the invention.

### Example 1: Film-coated tablet (not according to the claims)

| **ingredients** | **Amount (%)** |
|---|---|
| Linagliptin | 1.0 - 5.0 |
| Mannitol | 70.0 - 85.0 |
| Sodium starch glycolate | 0.1 - 5.0 |
| Povidone | 0.1 - 3.0 |
| Sodium stearyl fumarate | 1.0 - 5.0 |
| Coating | 1.0 - 6.0 |
| **Total** | **100.0** |

### Example 2: Preferred film-coated tablet (not according to the claims)

| **ingredients** | **Amount (%)** |
|---|---|
| Linagliptin | 2.7 |
| Mannitol | 82.1 |
| Sodium starch glycolate | 4.9 |
| Povidone | 2.9 |
| Sodium stearyl fumarate | 2.0 |
| Coating | 5.4 |
| **Total** | **100.0** |

The pharmaceutical formulations mentioned above are prepared by following these steps:
- mixing linagliptin, mannitol and sodium starch glycolate in a fluid bed dryer to prepare a powder mixture,
- solving povidone in water to prepare a granulation solution,
- granulating the powder mixture with the granulation solution using spray granulation method,
- drying the granules, preferably until they have less than 2% moisture by weight,
- sieving the granules and grinding them,
- adding sodium stearyl fumarate into the grinded granules and mixing them together to prepare the final mixture
- compressing the final mixture into tablets,
- coating these tablets with a film layer

Wet granulation method which is performed to granulate the powder mixture results in granules with a round, compact structure, excellent flow properties, a high bulk density and a narrow range of particle sizes. These properties make easier to process and enable the use of the selected excipients mentioned above. In addition to this, spray granulation with fluid bed technology enables to choose residual moisture and particle size. Consequently, the tablet dosage form has an enhanced uniformity and stability with a prolonged shelf-life.

## Claims

1. A solid oral pharmaceutical formulation comprising linagliptin or a pharmaceutically acceptable salt thereof, wherein the composition is free of microcrystalline cellulose, croscarmellose sodium and stearates; wherein the total amount of starch in the composition is less than 5% by weight of the total composition and wherein the solid oral pharmaceutical formulation further comprises polyvinyl alcohol in an amount of between 0.1-10% by weight of the total formulation.

2. The solid oral pharmaceutical formulation according to claim 1, wherein the composition comprises linagliptin in an amount of 0.1-15%, preferably 0.5-10%, more preferably 1-5% by weight of the composition.

3. The solid oral pharmaceutical composition according to any one of the preceding claims, wherein the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet or orally administrable film.

4. The solid oral pharmaceutical composition according to claim 3, wherein the composition is in the form of a film-coated tablet.

5. The solid oral pharmaceutical composition according to any one of the preceding claims, wherein the composition further comprises at least one pharmaceutically acceptable excipient selected from diluents, disintegrants, binders, lubricants or mixtures thereof.

6. The solid oral pharmaceutical composition according to claim 5, wherein the composition comprises at least one diluent which is selected from the group comprising lactose monohydrate, lactose, dibasic calcium phosphate, mannitol, spray-dried mannitol, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

7. The solid oral pharmaceutical composition according to claim 6, wherein the composition comprises one diluent which is mannitol.

8. The solid oral pharmaceutical composition according to claim 7, wherein the amount of mannitol is between 20-95%, preferably 40-90% and more preferably 70-85% by weight of the total composition.

9. The solid oral pharmaceutical composition according to claim 5, wherein the composition comprises at least one lubricant which is selected from the group comprising sodium lauryl sulphate, sodium stearyl fumarate, mineral oil, talc, polyethylene glycol, magnesium lauryl sulphate, fumaric acid, stearic acid, hydrogenated natural oils, silica, paraffin or mixtures thereof.

10. The solid oral pharmaceutical composition according to claim 9, wherein the composition comprises one lubricant which is sodium stearyl fumarate.

11. The solid oral pharmaceutical composition according to claim 10, wherein the amount of sodium stearyl fumarate is between 0.1-10%, preferably 1-5% by weight of the total composition.

12. The solid oral pharmaceutical composition according to any one of the preceding claims, wherein the composition is free of glidant.

## Patentansprüche

1. Eine feste orale pharmazeutische Formulierung, umfassend Linagliptin oder ein pharmazeutisch akzeptables Salz davon, wobei die Zusammensetzung frei von mikrokristalliner Cellulose, Croscarmellose-Natrium und Stearaten ist; wobei die Gesamtmenge an Stärke in der Zusammensetzung weniger als 5 Gew.-% der Gesamtzusammensetzung beträgt und wobei die feste orale pharmazeutische Formulierung ferner Polyvinylalkohol in einer Menge zwischen 0,1 - 10 Gew.-% der Gesamtformulierung umfasst.

2. Die feste orale pharmazeutische Formulierung nach Anspruch 1, wobei die Zusammensetzung Linagliptin in einer Menge von 0,1 - 15 Gew.-%, vorzugsweise 0,5 - 10 Gew.-%, noch bevorzugter 1 - 5 Gew.-% der Zusammensetzung umfasst.

3. Die feste orale pharmazeutische Zusammensetzung gemäß einem der vorstehenden Ansprüche, wobei die Zusammensetzung in Form einer beschichteten Tablette, dreischichtigen Tablette, zweischichtigen Tablette, mehrschichtigen Tablette, oral zerfallenden Tablette, Minitablette, Pellet, Zuckerpellet, Bukkaltablette, Sublingualtablette, Brausetablette, Tablette mit sofortiger Freisetzung, Tablette mit modifizierter Freisetzung, Filmtablette, magensaftlösliche Tablette, Pille, Kapsel, orales Granulat, Pulver, beschichtetes Perlen-System, Mikrosphäre, Tablette in Tablette, Einlagetablette, Dragee, Sachet oder oral verabreichbarer Film.

4. Die feste orale pharmazeutische Zusammensetzung gemäß Anspruch 3, wobei die Zusammensetzung in Form einer Filmtablette vorliegt.

5. Die feste orale pharmazeutische Zusammensetzung gemäß einem der vorstehenden Ansprüche, wobei die Zusammensetzung ferner mindestens einen pharmazeutisch akzeptablen Hilfsstoff umfasst, der aus Verdünnungsmitteln, Sprengmitteln, Bindemitteln, Gleitmitteln oder Mischungen davon ausgewählt ist.

6. Die feste orale pharmazeutische Zusammensetzung gemäß Anspruch 5, wobei die Zusammensetzung mindestens ein Verdünnungsmittel umfasst, das aus der Gruppe ausgewählt ist, die aus Lactose-Monohydrat, Lactose, dibasischem Calciumphosphat , Mannitol, sprühgetrocknetes Mannitol, Dextrose, Saccharose, Fructose, Maltose, Sorbit, Xylitol, Inositol, Kaolin, anorganische Salze, Calciumsalze, Polysaccharide, Dicalciumphosphat, Natriumchlorid, Dextrate, Lactitol, Maltodextrin, Saccharose-Maltodextrin-Gemisch, Trehalose, Natriumcarbonat, Natriumhydrogencarbonat, Calciumcarbonat oder Mischungen davon ausgewählt ist.

7. Die feste orale pharmazeutische Zusammensetzung gemäß Anspruch 6, wobei die Zusammensetzung ein Verdünnungsmittel umfasst, das Mannitol ist.

8. Die feste orale pharmazeutische Zusammensetzung nach Anspruch 7, wobei die Menge an Mannitol zwischen 20 - 95 Gew.-%, vorzugsweise zwischen 40 - 90 Gew.-% und noch bevorzugter zwischen 70 - 85 Gew.-% der Gesamtzusammensetzung beträgt.

9. Die feste orale pharmazeutische Zusammensetzung nach Anspruch 5, wobei die Zusammensetzung mindestens ein Gleitmittel umfasst, das aus der Gruppe ausgewählt ist, die aus Natriumlaurylsulfat, Natriumstearylfumarat, Mineralöl, Talk, Polyethylenglykol, Magnesiumlaurylsulfat, Fumarsäure, Stearinsäure, hydrierten natürlichen Ölen, Siliciumdioxid, Paraffin oder Mischungen davon besteht.

10. Die feste orale pharmazeutische Zusammensetzung nach Anspruch 9, wobei die Zusammensetzung ein Gleitmittel umfasst, das Natriumstearylfumarat ist.

11. Die feste orale pharmazeutische Zusammensetzung nach Anspruch 10, wobei die Menge an Natriumstearylfumarat zwischen 0,1 - 10 Gew.-%, vorzugsweise zwischen 1 - 5 Gew.-% der Gesamtzusammensetzung beträgt.

12. Die feste orale pharmazeutische Zusammensetzung nach einem der vorstehenden Ansprüche, wobei die Zusammensetzung frei von Gleitmitteln ist.

## Revendications

1. - Formulation pharmaceutique orale solide comprenant de la linagliptine ou un sel pharmaceutiquement acceptable de celle-ci, la composition étant exempte de cellulose microcristalline, de croscarmellose sodique et de stéarates ; la quantité totale d'amidon dans la composition étant inférieure à 5 % en poids de la composition totale, et la formulation pharmaceutique orale solide comprenant en outre de l'alcool polyvinylique dans une quantité d'entre 0,1 et 10 % en poids de la formulation totale.

2. - Formulation pharmaceutique orale solide selon la revendication 1, dans laquelle la composition comprend de la linagliptine à hauteur de 0,1-15%, de préférence de 0,5-10%, de façon davantage préférée de 1-5% en poids de la composition.

3. - Composition pharmaceutique orale solide selon l'une quelconque des revendications précédentes, dans laquelle la composition est sous forme de comprimé enrobé, comprimé tricouche, comprimé bicouche, comprimé multicouche, comprimé à désagrégation orale, mini-comprimé, pastille, pastille de sucre, comprimé buccal, comprimé sublingual, comprimé effervescent, comprimé à libération immédiate, comprimé à libération modifiée, comprimé pelliculé, comprimé à désintégration gastrique, pilule, capsule, granulé oral, poudre, système de billes enrobées, microsphère, comprimé-dans-comprimé, comprimé incrusté, dragée, sachet ou film administrable par voie orale.

4. - Composition pharmaceutique orale solide selon la revendication 3, dans laquelle la composition est sous la forme d'un comprimé pelliculé.

5. - Composition pharmaceutique orale solide selon l'une quelconque des revendications précédentes, dans laquelle la composition comprend en outre au moins un excipient pharmaceutiquement acceptable choisi parmi les diluants, les désintégrants, les liants, les lubrifiants ou leurs mélanges.

6. - Composition pharmaceutique orale solide selon la revendication 5, dans laquelle la composition comprend au moins un diluant qui est choisi dans le groupe comprenant le lactose monohydraté, le lactose, le phosphate de calcium dibasique, le mannitol, le mannitol séché par pulvérisation, le dextrose, le saccharose, le fructose, le maltose, le sorbitol, le xylitol, l'inositol, le kaolin, les sels inorganiques, les sels de calcium, les polysaccharides, le phosphate dicalcique, le chlorure de sodium, les dextrates, le lactitol, la maltodextrine, le saccharose-maltodextrine, le tréhalose, le carbonate de sodium, le bicarbonate de sodium, le carbonate de calcium ou leurs mélanges.

7. - Composition pharmaceutique orale solide selon la revendication 6, dans laquelle la composition comprend un diluant qui est le mannitol.

8. - Composition pharmaceutique orale solide selon la revendication 7, dans laquelle la quantité de mannitol est entre 20 et 95 %, de préférence entre 40 et 90 % et de façon davantage préférée entre 70 et 85 % en poids de la composition totale.

9. - Composition pharmaceutique orale solide selon la revendication 5, dans laquelle la composition comprend au moins un lubrifiant qui est choisi dans le groupe comprenant le lauryl sulfate de sodium, le fumarate de stéaryle et de sodium, l'huile minérale, le talc, le polyéthylène glycol, le lauryl sulfate de magnésium, l'acide fumarique, l'acide stéarique, les huiles naturelles hydrogénées, la silice, la paraffine ou leurs mélanges.

10. - Composition pharmaceutique orale solide selon la revendication 9, dans laquelle la composition comprend un lubrifiant qui est le fumarate de stéaryle et de sodium.

11. - Composition pharmaceutique orale solide selon la revendication 10, dans laquelle la quantité de fumarate de stéaryle et de sodium est entre 0,1 et 10 %, de préférence entre 1 et 5 % en poids de la composition totale.

12. - Composition pharmaceutique orale solide selon l'une quelconque des revendications précédentes, dans laquelle la composition est exempte d'agent glissant.
